# EUROPEAN PATENT SPECIFICATION

(11) **EP 4 313 108 B1**
(45) Date of publication and mention of the grant of the patent: **19.08.2026**
(21) Application number: 22713688.4
(22) Date of filing: 31.03.2022
(51) Int. Cl.: A61K 38/17, A61P 31/14, C07K 14/47

(54) **EZRIN PEPTIDE 1 FOR USE IN A METHOD OF TREATING POST COVID-19**
EZRIN-PEPTID 1 ZUR VERWENDUNG IN EINEM VERFAHREN ZUR BEHANDLUNG VON POST COVID-19
PEPTIDE D'EZRINE 1 DESTINÉ À ÊTRE UTILISÉ DANS UN PROCÉDÉ DE TRAITEMENT DE SYNDROME POST-COVID-19

(30) Priority: 31.03.2021 WO PCT/EP2021/058459; 01.10.2021 EP 21200476
(43) Date of publication of application: 07.02.2024
(73) Proprietor: Pantapharm AG, 37115 Duderstadt (DE)
(72) Inventor: NESSELHUT, Thomas, 37115 Duderstadt (DE); NESSELHUT, Jan, 37115 Duderstadt (DE); OSMERS, Rüdiger, 37115 Duderstadt (DE)
(74) Representative: Meissner Bolte Partnerschaft mbB
(86) International application number: PCT/EP2022/058622
(87) International publication number: WO 2022/207818

(56) References cited:
- WO-A2-2021/198346
- PETERS J HINRICH ET AL: "Akuttherapie von Viruskrankheiten", 24 March 2020 (2020-03-24), pages 1 - 3, XP055813111, Retrieved from the Internet <URL:https://jimdo-storage.global.ssl.fastly.net/file/79973013-b216-4ade-bc96-b1a4ecfb8a3b/Peters_Akuttherapie_von_Viruskrankheiten.pdf> [retrieved on 20210611]
- BESSLER WOLFGANG: "Arbeitsgruppe Vakzine", 24 March 2020 (2020-03-24), pages 1 - 23, XP055813145, Retrieved from the Internet <URL:http://web.archive.org/web/20200601141608/https://sites.google.com/site/alsanafreiburg/home/ak-vakzine> [retrieved on 20210611]
- HOLMS R. D. ET AL: "Review of Russian ezrin peptide treatment of acute viral respiratory disease and virus induced pneumonia; a potential treatment for covid-19", 12 April 2020 (2020-04-12), pages FP,1-41, XP055813400, Retrieved from the Internet <URL:https://www.researchgate.net/publication/340600041_Review_of_Russian_ezrin_peptide_treatment_of_acute_viral_respiratory_disease_and_virus_induced_pneumonia_a_potential_treatment_for_covid-19> [retrieved on 20210614], DOI: 10.13140/RG.2.2.10214.57925
- MILLET JEAN KAORU ET AL: "Ezrin Interacts with the SARS Coronavirus Spike Protein and Restrains Infection at the Entry Stage", vol. 7, no. 11, 21 November 2012 (2012-11-21), pages e49566, XP055813049, Retrieved from the Internet <URL:https://storage.googleapis.com/plos-corpus-prod/10.1371/journal.pone.0049566/1/pone.0049566.pdf?X-Goog-Algorithm=GOOG4-RSA-SHA256&X-Goog-Credential=wombat-sa@plos-prod.iam.gserviceaccount.com/20210611/auto/storage/goog4_request&X-Goog-Date=20210611T113929Z&X-Goog-Expires=86400&X-Goog-SignedHeaders=h> DOI: 10.1371/journal.pone.0049566
- ANONYMOUS: "Ezrin Peptide (HEP-1) for Treatment of Coronavirus Disease (COVID-19) Infection", 13 November 2020 (2020-11-13), pages 1 - 4, XP055813549, Retrieved from the Internet <URL:https://clinicaltrials.gov/ct2/show/NCT04627233> [retrieved on 20210614]
- VAN KAMPEN JEROEN J.A. ET AL: "Shedding of infectious virus in hospitalized patients with coronavirus disease-2019 (COVID19): duration and key determinants", MEDRXIV, 9 June 2020 (2020-06-09), XP055835315, Retrieved from the Internet <URL:https://www.medrxiv.org/content/10.1101/2020.06.08.20125310v1.full.pdf> [retrieved on 20210826], DOI: 10.1101/2020.06.08.20125310
- NALBANDIAN ANI ET AL: "Post-acute COVID-19 syndrome", NATURE MEDICINE, NATURE PUB. CO, NEW YORK, vol. 27, no. 4, 22 March 2021 (2021-03-22), pages 601 - 615, XP037424502, ISSN: 1078-8956, [retrieved on 20210322], DOI: 10.1038/S41591-021-01283-Z
- THALLER ARNO: "Was steht auf dem Spielplan im Welt-Theater ? Corona", 25 November 2021 (2021-11-25), pages 1 - 10, XP055923226, Retrieved from the Internet <URL:https://jimdo-storage.global.ssl.fastly.net/file/73db2b70-e5c1-471f-bb9e-13d7830f1501/Corona-Theater%2011-2021.pdf> [retrieved on 20220519]

## Description

The present invention concerns Ezrin peptide 1 for use in a method of treating post COVID-19 syndrome.

Coronavirus disease 2019 (COVID-19) is caused by a novel coronavirus called SARS-CoV-2. The acronym "SARS" stands for severe acute respiratory syndrome. An outbreak of COVID-19 in the Hubei province (China) at the end of 2019 is spreading globally and is impacting the health of people and of the economy worldwide. As of March 30, 2020, COVID-19 has been confirmed in about 750,000 people worldwide, carrying a mortality of approximately 3-5%, compared with a mortality rate of less than 1% from influenza. Most diagnosis relies on PCR using specimens from the respiratory tract.

On 11 March 2020, the Director-General of WHO declared the spate of infections caused by SARS-CoV-2 (COVID-19) a pandemic. The novel coronavirus SARS-CoV-2 causes flu-like symptoms such as dry cough, fever, a runny nose and fatigue. There have also been reports of an itchy throat, headaches, joint pains, nausea, diarrhoea and shivering. The virus spreads from person to person. Droplet infection is the main mode of transmission. Transmission can take place directly, from person-to-person, or indirectly through contact between hands and the mucous membranes of the mouth, the nose or the conjunctiva of the eyes. There have been reports of persons who were infected by individuals who had only shown slight or non-specific symptoms of disease. The percentage of asymptomatic cases is unclear. It is currently thought that an infected person can go up to 14 days before noticing any symptoms. According to WHO, the incubation period is, on average, five to six days.

Among the cases reported to date, in three out of five cases infection was mild with symptoms, e.g. dry cough and fever less than 39.5°C, for about 1 week. This means that not all diseases that follow a SARS-CoV-2 infection take a serious course and require treatment. Unlike other acute infectious diseases progressing to sepsis, the severe courses of COVID-19 seemingly show prolonged progression from onset of first symptoms to life-threatening deterioration of (primarily) lung function including difficult breathing and pneumonia. Severe acute respiratory distress syndrome (ARDS) reflects the hallmark of a critical course of the disease. Treatment of the infection depends on the severity of the disease presentation (e.g. administering oxygen, maintaining fluid balance, if necessary administering antibiotics to combat bacterial co-infections) and also includes the treatment of relevant underlying chronic illnesses. Current management of COVID-19 is supportive and ARDS is the leading cause of mortality.

Accumulating evidence suggests that a subgroup of patients with severe COVID-19 might have a cytokine storm syndrome which leads to hyper-inflammation and irremediable lung tissue damage. It has been reported that a cytokine profile resembling sHLH (secondary haemophagocytic lymphohistiocytosis) is associated with COVID-19 disease severity, characterized by increased interleukin (IL)-2, IL-7, granulocyte-colony stimulating factor, interferon-γ inducible protein 10, monocyte chemo-attractant protein 1, macrophage inflammatory protein 1-α, and tumour necrosis factor-α. Predictors of fatality from a recent retrospective, multicentre study of 150 confirmed COVID-19 cases in Wuhan, China, included elevated ferritin (mean 1297·6 ng/ml in non-survivors vs 614·0 ng/ml in survivors; p<0·001) and IL-6 (p<0·0001), suggesting that mortality might be due to virally driven hyper-inflammation (Mehta et al., The Lancet, Vol. 395, p. 1033-1034 (March 2020)).

During the course of the COVID-19 pandemic, it has also become apparent that a large number of patients infected with SARS-CoV-2 suffer from late/long-term sequelae long after the acute infection has subsided. Although affected individuals have recovered from acute COVID-19 symptoms or may not have developed acute COVID-19 symptoms during infection, they are by no means healthy. Instead, they suffer long-term persisting multiple symptoms. These long-term symptoms are also termed post-COVID-19 syndrome, post COVID-19, post-acute COVID-19 (syndrome), long COVID (syndrome) or late COVID (syndrome). Post-COVID-19 syndrome can affect nearly every organ system. Symptoms of post-COVID-19 syndrome include in particular the following disorders or conditions:
- Fatigue (most common);
- Disorders of the respiratory system, such as reduced lung function, dyspnea, shortness of breath, long lasting/persistant cough;
- Cardiovascular disorders;
- Metabolic disorders;
- Gastrointestinal disorders or gastrointestinal symptoms, such as diarrhea, vomiting, gastroesophageal reflux disease;
- Disorders of the nervous system and neurocognitive disorders, such as headaches, needle pains in arms and legs;
- Narcolepsy;
- Dysautonomia (pure autonomic failure, PAF);
- Loss of taste and smell;
- Sleep disorders;
- Mental health disorders such as cognitive dysfunction, inability to concentrate, loss of memory, in particular loss of short-term memory;
- Musculoskeletal pain, such as pain in the lumbar region and muscle weakness;
- Spondylitis;
- Intermittent fever;
- Anxiety and depression;
- Skin rash;
- Kidney disorders, such as acute kidney injury, and chronic kidney disease;
- Blood clotting, such as deep vein thrombosis and pulmonary embolism;
- Endocrine sequelae, such as new onset of diabetes, greater difficulty controlling an existing diabetes mellitus, subacute thyroiditis and bone demineralization and
- New onset of hypertension.

The symptoms can persist for months and can worsening within time. Existing disorders can be activated and worsening as well (see: https://www.bhf.org.uk/informationsupport/heart-matters-magazine/news/coronavirus- and-your-health/long-covid). The post-COVID-19 syndrome can appear in people who had a mild or moderate initial infection as well as those who were admitted to hospital with more severe infection. Even children can suffer from post-COVID-19 syndrome. Post COVID-19 syndrome has been discussed e. g. in the following references:
*1.* Al-Aly Z, Xie Y, Bowe B (June 2021), Nature. 594 (7862): 259-64;
2. Lopez-Leon S et al. (August 2021), Scientific Reports. 11 (1): 16144;
3. Davis HE et al. (July 2021), E Clinical Medicine. 38:101019;
4. Tenforde MW et al. (July 2020), MMWR. Morbidity and Mortality Weekly Report. 69 (30): 993-98.
5. Yelin D, Wirtheim E, Vetter P, Kalil AC, Bruchfeld J, Runold M, et al. (October 2020), The Lancet. Infectious Diseases. 20 (10): 1115-17;
6. Ludvigsson JF (March 2021), Acta Paediatrica. 110(3): 914-21;
7. A. Nalbandian et al. in Nature Medicine, 27 (2021) pp 601-615.

A. Nalbandian et al. assume that potential mechanisms contributing to the pathophysiology of post-acute COVID-19 include (1) virus-specific pathophysiologic changes; (2) immunologic aberrations and inflammatory damage in response to the acute infection, such as perivascular inflammation; and (3) expected sequelae of post-critical illness.

There is an urgent need for an effective treatment of post-COVID-19 syndrome. Currently, the focus is on vaccinating the population to reduce or even prevent the risk of infection with Sars-CoV-2 and the resulting sequelae. This does not guarantee complete protection against infection with Sars-CoV-2. In view of the partly very infectious Sars-CoV-2 variants and the partly unwillingness of the population to be vaccinated and the partly lack of availability of vaccine in emerging countries, a widespread immunization of the world population is very difficult. The development of new therapeutics for COVID-19, including antiviral agents, is difficult and it may take at least several months, in the worst case more than 1 year, until a suitable new medication will be available. Until then there is an urgent need for the treatment of post-COVID-19 syndrome.

H. J. Peters et al. "Akuttherapie von Virkuskrankheiten (2020-03-24), pages 1-3, XP055813111 (retrieved from the Internet: hhttps://jimdo-storage-global.ssl.fastly.net/file/z9973013-b216-4ade-bc96-b1a4ecfb8a3b/Peters_Akuttherapie_von_Viruskrankheiten.pdf) discuss the use of GEPON, a peptide from the humend immune systeme dreivied from the surface molecule Ezrin, as an antiviral. They suggest to test GEPON against COVID-19 in acute therapy.

R. D. Holmes et al: "Review of Russiona ezrin peptide treatment of acute viral respiratory disease and virus induced pneumonia; a potential treatment for void 19" (2020-04-12) page FP, 1-41 XP055813400 DOI: 10.13140/RG.2.2.10214.57925, suggest that oral, nasal and vapour Ezrin Peptide therapy should amplify antiviral immunity and reduce inflammatory events in COVID-19 patients.

The inventors have found out that Ezrin peptide 1 or pharmaceutical compositions comprising Ezrin peptide 1 and at least one pharmaceutically acceptable carrier are suitable for the treatment of the post-COVID-19 syndrome. The inventors have also found out that analogues of Ezrin peptide 1 of the formula (I) as described or pharmaceutical compositions comprising such an analogue of Ezrin peptide 1 and at least one pharmaceutically acceptable carrier are suitable for the treatment of the post-COVID-19 syndrome. The inventors have also found out that combinations of Ezrin peptide 1 and an analogue of Ezrin peptide 1 of the formula (I) as described herein, as well as pharmaceutical compositions comprising a combination of Ezrin peptide 1 and an analogue of Ezrin peptide 1 of the formula (I) as described herein and at least one pharmaceutically acceptable carrier are suitable for the treatment of the post-COVID-19 syndrome.

Administration of Ezrin peptide 1 and/or of an analogue of Ezrin peptide 1 of the formula (I) as described herein or combinations thereof to an individual suffering from post-COVID-19 syndrome results in an improvement of the symptoms of post-COVID-19 syndrome or even the complete curing from post-COVID-19 syndrome. Moreover, prophylactic treatment with Ezrin peptide 1 and/or with an analogue of Ezrin peptide 1 of the formula (I) of an individual who has recovered from COVID-19 but has not yet developed post-COVID-19 syndrome results in a reduced risk of post-COVID-19 syndrome.

Ezrin protein, also known as cytovillin or villin-2, is a protein encoded in humans by the EZR gene. Ezrin peptide 1 for use according to the present invention comprises or is a pharmaceutical tetradecapeptide NH2_Thr-Glu-Lys-Lys-Arg-Arg-Glu-Thr-Val-Glu-Arg-Glu-Lys-Glu_COOH (sequence ID No. 1: TEKKRRETVERKEKE), comprising 14 amino acid residues. Ezrin peptide 1 is also known as HEP-1 peptide or human Ezrin peptide one (TEKKRRETVEREKE). Ezrin peptide 1 is immune-modulatory and shows an anti-viral effect in cell cultures (R. Ataullakhanov et al.; Antiviral Mechanisms of the drug 'Gepon': Modulation of Cytokine Gene Transcription in a J-96 Human Cell Line. Eksp Klin Gastroenterol. 2005;(1):14-9, 106). An explanation of the anti-viral effect was that the docking of the virus to the target cell could be prevented. In addition, it was shown in cell cultures that it has an effect on the inflammatory cytokine production and, thus, has an influence on the inflammatory stress. It reduces the production of pro- and inflammatory cytokins (e.g. IL-1, IL-6, TNF-alpha) but induces the synthesis of alpha- and beta interferons. Ezrin peptide 1 was developed for the treatment of HIV-infection (WO 95/33768 A1). Ezrin peptide 1 is known to have anti-viral hepatitis C biological activity and can be used for the treatment of the patients with hepatitis C (WO 2004/067024 A2). It has been additionally reported that Ezrin peptide 1 has antiulcer biological activity and can be used for the treatment of ulcer diseases of the gastrointestinal tract (WO 2007/060440).

The analogues of Ezrin peptide 1 are tetradecapeptides, which are of the following general formula:

NH2_X¹-Glu-Lys-Lys-Arg-Arg-Glu-Thr-Val-Glu-Arg-Glu-X²-X³_COOH (I)

where X¹, X² and X³ are identical or different and are non-polar amino acid residues, where the amino acids are in particular selected from the group consisting of glycine, alanine, valine, leucine, methionine, isoleucine, proline, phenylalanine, tryptophan and combinations thereof. A particular example of such an analogues of Ezrin peptide 1 is the compound of formula (I), where X¹, X² and X³ are glycine residues, i.e. the compound of sequence ID No. 2: GEKKRRETVERKEGG or NH2_Gly-Glu-Lys-Lys-Arg-Arg-Glu-Thr-Val-Glu-Arg-Glu-Gly-Gly_COOH (SEQ ID NO:2), respectively. The analogues of Ezrin peptide 1 of the formula (I) are known from US 2016/0346383.

The peptides for use according to the present invention, i.e. Ezrin peptide 1 and its analogues of formula (I) can be synthesized by peptide synthetic chemistry well known in the art, e.g. from US 2016/0346383 and the references cited therein. For example, the peptides of the invention can be synthesized by liquid-phase synthesis using standard procedure or by solid-phase synthesis or by combinations thereof. When solid-phase synthesis is employed, then a solid phase is used, such as polystyrene resin or polyamide resin, or PEG hybrid polystyrene resin, or resin based on PEG. Different protective groups are used during the synthesis, for example, N-terminal protecting groups, t-Boc or FMOC protective groups. In some instances, fragments may be synthesized using solid-state methods and then coupled together in solution. Peptides can be synthesized from the carbonyl group side to amino group side of the amino acid chain in this method, although peptides are synthesized in the opposite direction in cells. In such methods, an amino-protected amino acid is bound to a substrate bead (i.e. a resin bead) forming a covalent bond between the carbonyl group and the resin. The amino group is then de-protected and reacted with the carbonyl group of the next amino-protected amino acid. The cycle is repeated as often as required in order to form the desired peptide chain. The synthesized peptide is then cleaved from the bead at the end of the procedure. The protecting groups for the amino groups mostly used in this peptide synthesis are 9-fluorenylmethyloxycarbonyl group ("Fmoc") and t-butyloxycarbonyl ("Boc"). The Fmoc group is removed from the amino terminus with base while the Boc group is removed with acid. Moreover, benzyloxycarbonyl (Z) groups or allyloxycarbonyl (Alloc) protective groups, or photo-removable (lithographic) protective groups, or side group protection technique may be employed. Peptide products are purified by HPLC separation or by any other purification method. Peptide structure is confirmed by amino acid analysis, mass spectrometry, and high performance liquid chromatography data..

"Subject" or "patient" or "individual" as used herein refers to humans having previously been infected with SARS-CoV-2. In particular, this term refers to humans who have previously been tested positive for SARS-CoV-2. These subjects may be affected by Coronavirus disease 2019 (COVID-19) but they may not necessarily have suffered from COVID-19, i.e. they may not necessarily have developed the symptoms associated with COVID-19.

Here and in the following, the terms post-COVID-19 syndrome, post-acute COVID-19 syndrome, long COVID or late COVID are used synonymously.

Although the development of post-COVID-19 syndrome can be avoided, or at least the risk of developing post-COVID-19 syndrome or a severe form thereof may be reduced, by administration of Ezrin peptide 1 and/or its analogues of the formula (I) during the acute phase of COVID-19 infection, amelioration or even cure of long COVID can also be achieved by administration of Ezrin peptide 1 and/or its analogues after the acute infection has subsided, even weeks or months thereafter. In other words, amelioration or even cure of post-COVID-19 syndrome and its pathological symptoms can be achieved by administration of Ezrin peptide 1 and/or its analogues during post-COVID-19 syndrome or before the symptoms of post-COVID-19 syndrome have been emerged. To this end, Ezrin peptide 1 and/or its analogues will typically be administered to an individual suffering from post-COVID-19 syndrome. Ezrin peptide 1 and/or its analogues may also be administered prophylactically to an individual who is expected to develop a post-COVID-19 syndrome, e.g. because the individual had previously suffered from COVID-19 or because they had been infected with SARS-CoV-2 without developing symptoms associated with COVID-19.

The Ezrin peptide 1 and/or its analogue of the formula (I), such as the pharmaceutical composition comprising Ezrin peptide 1 and/or an analogue thereof may be administered to the subject prior to, during and after symptoms associated with post-COVID-19 are present. Ideally, Ezrin peptide 1 and/or an analogue thereof as described herein is administered before or immediately after the onset of at least one symptom associated with post-COVID-19 syndrome. Frequently, this is not possible. Therefore, Ezrin peptide 1 and/or its analogue of the formula (I) is usually administered several days or weeks or even months after at least one symptom associated with post-COVID-19 syndrome has emerged. As a general rule: The earlier, the better.

The dosage may be administered in one portion daily or in several partial doses over the day or every second day. The administration should take place during the course of the infection and be maintained until the last one of the symptoms associated with post-COVID-19 syndrome has disappeared for at least one day, or better, for at least two days. As mentioned above, Ezrin peptide 1 and/or an analogue thereof, such as the pharmaceutical composition comprising Ezrin peptide 1 and/or an analogue thereof may also be administered prophylactically.

The dosage of Ezrin peptide 1 and its analogues of the formula (I) is not particularly critical, as Ezrin peptide 1 and its analogues are not toxic even at high dosages of 50 mg per kg of body weight per day or higher. Preferably, Ezrin peptide 1 and its analogues are administered to an adult human in an amount of at least 0.05 milligrams per day (mg/d), in particular at least 0.1 mg/d, e.g. in an amount of 0.05 to 5 mg/d, in particular 0.1 to 2 mg/d or 0.1 to 1 mg/d or 0.1 to 0.5 mg/d. Typically, for parenteral administrations lower dosages will be required than for oral or rectal administration. To achieve a rapid relief of the symptoms of post-COVID-19 syndrome, it may also be reasonable to administer at least 0.5 mg per day or at least 1 mg per day, e.g. in the range of 0.5 to 5 mg/d, but in other cases a lower dosage may be suitable. It is also possible to start with a higher dosage, e. g. in the range of 0.5 to 5 mg/d, more preferably 0.5 to 3 mg/d and then continue with a lower dosage of e.g. 0.05 to 0.5 mg/d or 0.1 to 0.5 mg/day. A single administration of Ezrin peptide 1 and/or one of its analogues of the formula (I) may be sufficient to ameliorate or even cure the symptoms of a post-COVID-19 syndrome. Preferably, the treatment with Ezrin peptide 1 and/or one of its analogues of the formula (I) of the individual suffering from post-COVID-19 syndrome is continued for a time period of several days or weeks until the pathological symptoms have significantly improved or preferably until they have completely subsided. Depending on the severity of the symptoms, this time period is typically in the range of 5 days to 10 weeks and in particular in the range of 10 days to 8 weeks.

The dosage of Ezrin peptide 1, its analogues of the formula (I) or the pharmaceutical composition containing the same is determined by the physician on the basis of patient-specific parameters, such as age, weight, sex, severity of the symptoms, etc. The terms "dosage regimen" or "mode of treatment" refer to a timely sequential or simultaneous administration of the Ezrin peptide 1 or its analogue or a combination thereof, and any other optional pharmaceutically active substance. This means that the components may be provided in a unit dosage form with physical contact to each other (e.g. one single tablet or solution) or as separate entities (e.g. two tablets or solutions) to be taken simultaneously or with a certain time difference. This time difference may be in the range of 0.5 hour and 2 days, preferably between 1 hour and 24 hours.

The pharmaceutical compositions for use according to the present invention may comprise other pharmaceutically active substances, such as anti-viral, anti-bacterial, analgesic and/or anti-inflammatory substances. In a preferred embodiment, the pharmaceutical composition may also contain immune-stimulatory agents. Examples for anti-viral substances are acyclovir, ribavirin, valaciclovir, oseltamivir, remdesivir or zanamivir. Examples for anti-bacterial substances are antibiotics including those of the classes aminoglycosides, ansamycins, carbacephmes, cephalosporins, glycopeptides, macrolides, penicillins, polypeptides, quinolones, sulfonamides, tetracyclines, glycycyclines, oxazolidinones, amphenicols, pleuromutilins, lincosamides, streptogramins, steroid antibacterials, cyclopeptides lipopeptides, and mixtures thereof.

The term "about" as used herein, means in quantitative terms plus or minus 5%, of the respective value given.

"Pharmaceutically acceptable" is meant to encompass any carrier, which does not interfere with the effectiveness of the biological activity of the active ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and can be administered to the subject at an effective dose. Additional pharmaceutically compatible carriers can include gels, bioadsorbable matrix materials, implantation elements containing the therapeutic agent, or any other suitable vehicle, delivery or dispensing means or material(s).

The pharmaceutical composition may be prepared and administered in any dosage form suitable for administration to the subject's body. It is formulated to be compatible with its intended route of administration. Examples of routes of administration include parenteral, e.g. intravenous, intradermal, intramuscular, intra-arterial or subcutaneous administration, and oral administration. Suitable routes of administration are also inhalation and intranasal administration. Another suitable route is rectal administration. Preferably, the administration route is subcutaneous.

When administered subcutaneously, the pharmaceutical composition is preferably injected into well perfused tissue, for example subumbellic tissue, for example subumbellically laterally at the height of the iliac spine.

The pharmaceutical composition may be formulated using any convenient adjuvant and/or physiologically acceptable diluents. The type of formulation depends on the route of administration in a known manner.

In a preferred embodiment, the formulation may be prepared as a tablet, a lozenge, a liquid, gel, a suspension, an emulsion or a solution.

Solutions or suspensions used for parenteral, in particular subcutaneous, application can include the following components: a sterile diluent such as water for injection, (physiological) saline solution, phosphate buffered saline, fixed oils, polyethylene glycols, glycerine, propylene glycol, or other synthetic solvents; antibacterial agents such as benzyl alcohol or methyl parabens; antioxidants such as ascorbic acid or sodium bisulfate; chelating agents such as ethylenediamine tetraacetic acid (EDTA); buffers such as acetates, citrates, or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for parenteral administration are injectables. For the preparation of injectables sterile aqueous solutions, dispersions or emulsions are used. Sterile powders for the extemporaneous preparation of sterile injectable solutions or dispersions may also be used. For parenteral administration, suitable carriers include physiological saline, bacteriostatic water, Cremophor^{®} EL (BASF, Parsippany, N.J.), or phosphate buffered saline (PBS). In all cases, the composition must be sterile and should be fluid to the extent that easy syringability exists. It should be stable under the conditions of manufacture and storage and must be preserved against the contaminating action of microorganism such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganism can be achieved by various antibacterial and antifungal agents, for example, parabens, chlorobutanol, phenol, ascorbic acid, thimerosal, and the like. According to embodiments, isotonic agents, for example, sugars, polyalcohols such as manitol, sorbitol, or sodium chloride in the composition are added. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by sterilization, e.g. by filtration or heat sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preparation is prepared by vacuum drying or freeze-drying, which yields a powder of the active ingredient plus any additional desired ingredient from a previously sterile-filtered solution thereof.

In a further preferred embodiment, the formulation for use according to present invention is suitable for oral administration. Oral compositions generally include inert diluents or edible carriers. For the purpose of oral therapeutic administration, the active compound can be incorporated into excipients and can be used in the form of tablets, troches, chewie, lozenges, gel caps, soft gel or capsules, e.g. gelatin capsules. Oral compositions can also be prepared using a fluid carrier for use as a mouthwash. Pharmaceutically compatible binding agents or adjuvant materials can be included as part of the composition. The tablets, pills, capsules, troches, lozenges and the like can contain any of the following ingredients, or compounds of a similar nature: a binder such as microcrystalline cellulose, gum tragacanth or gelatin; an excipient such as starch or lactose, a disintegrating agent such as alginic acid, primogel (sodium starch glycolate), or corn starch; a lubricant such as magnesium stearate; a glidant such as colloidal silicon dioxide; a sweetening agent such as sucrose or saccharin.

The formulation for use according to present invention may include any additional component as desired, as long as such components do not substantially erode the effectiveness of the Ezrin peptide 1. Of course, the additional components are typically pharmaceutically acceptable. Such components may include, for example, thickeners, sweeteners, flavorants, fragrances, additional pharmaceuticals, glycine, mannitol, silicone dioxide, silica gels, binders, vitamins, carriers, such as, for example, glycerin, starches, celluloses, chitins, water, alcohol, and minerals.

Pharmaceutical compositions suitable for inhalation include any formulation that can be converted into an aerosol by means of an inhalation system, including meter dosed inhalers, dry powder inhalers, soft-mist inhalers and nebulizers. In particular, the pharmaceutical compositions for inhalation are suitable for being converted into an aerosol having an particle size of at most 10 µm, in particular at most 5 µm. Suitable compositions include solutions, suspension and also dry powders. Depending on the type of composition, they may contain pharmaceutically acceptable solid or liquid carriers, and/or pharmaceutically acceptable excipients, including water, physiological saline, bacteriostatic water, PBS, amino acids including, leucine, isoleucine, glycine, and methionine; surfactants such as polysorbates, and sugars, including mannitol, lactose, trehalose, raffinose and the like. Particularly suitable are solutions of Ezrin peptide 1 and/or an analogue thereof in physiologic saline. Pharmaceutical compositions suitable for intranasal administration include any formulation mentioned for inhalation, in particular liquid formulations such as solutions of Ezrin peptide 1 and/or an analogue thereof in physiologic saline.

Fillers, carriers, preservatives, and stabilizers, which are usually used by persons skilled in drug delivery technology, may be used as an acceptable carrier or filler for preparation of the provided pharmaceutical compositions. For injections, distilled water or physiologic saline are predominantly used.

Pharmaceutical compositions suitable for rectal administration are in particular suppositories.

### Examples

### Example 1: 57 year old male patient

Risk factor/pre-existing illnesses: none
Course of long Covid-19 condition:

| | |
|---|---|
| 06.05.2020: | acute COVID-19 disease (PCR-test positive) |
| End of May 2020 | no acute COVID-19 disease symptoms, but since then the patient was suffering from LONG-COVID-19 symptoms with Fatigue-syndrome, reduced mental ability, reduced lung function and low ability to walk (20 meters) without developing breath problems |
| 22.01.2021: | onset of therapy with injection of HEP-1: 0.2 mg s.c. |
| | continuing the daily treatment with HEP-1 (0.2 mg s.c. per day) until March 10^{th} 2021 |
| 29.01.2021: | remarkable improvement of the mental ability (short memory) and physical strength (walking distance no longer restricted). |
| 10.03.2021 | complete recovery |

### Example 2: 41 year old male patient

Risk factor/pre-existing illnesses: ankylosis spondylitis for 30 years
Course of long Covid-19 condition:

| | |
|---|---|
| 11.02.2020: | acute COVID-19 disease (PCR-test positive) |
| Beginning of March2020 | no acute COVID-19 disease symptoms, but since then the patient was suffering from LONG-COVID-19 symptoms with Fatigue-syndrome, reduced mental ability, reduced lung function with chronical cough, swelling of the scull, chronic headaches, increased pain in the lumbar region, worsening of the spondylitis and edema in whole body |
| 26.02.2021: | onset of therapy with injection of HEP-1: 0.2 mg s.c. |
| | continuing the daily treatment with HEP-1 (0.2 mg s.c. per day) until March 17^{th} 2021 |
| 04.03.2021: | remarkable improvement of the mental ability (short memory) and physical strength (walking distance no longer restricted). |
| 17.03.2021 | complete recovery |

### Example 3: 52 year old male patient

Risk factor/pre-existing illnesses: none
Course of long Covid-19 condition:

| | |
|---|---|
| 02.02.2021: | acute COVID-19 disease (PCR-test positive) |
| 24.02.2021 | no acute COVID-19 disease symptoms, but since then the patient was suffering from LONG-COVID-19 symptoms with Fatigue-syndrome, reduced mental ability (short memory), reduced lung function with dyspnea, low ability to walk (10 meters) without developing breath problems and narcolepsy |
| 29.03.2021: | onset of therapy with injection of HEP-1: 0.2 mg s.c. |
| | continuing the daily treatment with HEP-1 (0.2 mg s.c. per day) until April 24^{th} 2021 |
| 15.04.2021: | remarkable improvement of the mental ability (short memory) and physical strength (walking distance no longer restricted). |
| 23.04.2021 | complete recovery |

### Example 4: 51 year old male patient

Risk factor/pre-existing illnesses: no history of severe illness
Course of long Covid-19 condition:
   Onset of acute COVID-19 disease (PCR-test positive) in October 2020 with fever, headache, loss of taste and smell, fatigue syndrome and pneumonia. After 10 days fever disappeared and smell and taste came back to normal. The fatigue syndrome persisted from January 2021 until start of treatment in February 2021. The patient was treated by administering 0.2 mg s.c. of HEP-1 and the treatment was continued by daily treatment with HEP-1 (0.2 mg s.c. per day) for 10 days. Thereafter, the patient was free of any symptoms.

### Example 5: 47 year old male patient

Risk factor/pre-existing illnesses: no history of severe illness

Onset of acute COVID-19 disease (PCR-test positive) in January 2021 with characteristic symptoms including, inter alia, fatigue syndrome and cognitive disorder. Two weeks after the acute phase of the disease, the PCR test was negative. However, the fatigue syndrome and cognitive disorder persisted. At 26.02.2021 therapy of the patient by daily injection of HEP-1 (0.2 mg s.c per day) was begun. On 12.03.2021, the patient was free of any symptoms.

### Example 6: 44 year old male patient

### Risk factor/pre-existing illnesses: Asthma

In August 2020, the patient was hospitalized because of COVID-19 infection. After two weeks, the patient was discharges from the hospital. Beginning January 2021 typical long COVID-19-symptoms as nausea, acid reflux, chronical bronchitis, headaches, stomach-aches and fatigue syndrome appeared. Onset of HEP-1 therapy was 13.10.2021. The first 10 days, the dose of HEP-1 was 2 mg s.c. per day followed 30 days with 0.2 mg s.c. per day. After 3 weeks of treatment the patient became cured.

### Example 7: 20 year old male patient

### Risk factor/pre-existing illnesses: no history of severe illness

In February 2021, the patient suffered from COVID-19 infection (PCR-test positive) with characteristic symptoms. A hospitalization was not indicated. The acute phase lasted for weeks. In April 2021, the patient developed typical long COVID-19-syndrome with fatigue, cognitive disorder and narcolepsy. At the end of July the patient was treated by daily injection of HEP-1 (0.2 mg s.c per day). After 11 days of treatment, the symptoms completely disappeared.

## Claims

1. A compound selected from the group consisting of Ezrin peptide 1 comprising the amino acid sequence NH2_Thr-Glu-Lys-Lys-Arg-Arg-Glu-Thr-Val-Glu-Arg-Glu-Lys-Glu_COOH (SEQ ID NO:1), the analogue of Ezrin peptide 1 of the formula (I) and combinations of Ezrin peptide 1 and the analogue of Ezrin peptide 1 of the formula (I),
NH2_X¹-Glu-Lys-Lys-Arg-Arg-Glu-Thr-Val-Glu-Arg-Glu-X²-X³_COOH (I)
where X¹, X² and X³ are identical or different and are non-polar amino acid residues, where the amino acids are in particular selected from the group consisting of glycine, alanine, valine, leucine, methionine, isoleucine, proline, phenylalanine, tryptophan and combinations thereof,
for use in a method of treating post COVID-19 syndrome in a subject.

2. The compound for use according to claim 1, which is formulated for subcutaneous administration.

3. The compound for use according to claim 1, which is formulated for oral administration.

4. The compound for use according to claim 1, which is formulated for inhalation or for intranasal administration.

5. The compound for use according to any one of claims 1 to 4, which it is administered daily.

6. The compound for use according to any one of claims 1 to 5, which it is administered at a dosage of at least 0.05 mg per day.

7. The compound for use according to any one of claims 1 to 6, which it is administered for a period of at least 5 days at a dosage in the range of 0.05 to 5 mg per day.

8. The compound for use according to any one of claims 1 to 7, which is Ezrin peptide 1 comprising the amino acid sequence NH2_Thr-Glu-Lys-Lys-Arg-Arg-Glu-Thr-Val-Glu-Arg-Glu-Lys-Glu_COOH (SEQ ID NO:1).

9. The compound for use according to any one of claims 1 to 7, which is the analogue of Ezrin peptide 1 of the formula (I) as defined in claim 1.

10. The compound for use according to any one of claims 1 to 7, which is a combination of Ezrin peptide 1 and the analogue of Ezrin peptide 1 of the formula (I) as defined in claim 1.

11. A pharmaceutical composition comprising at least one compound selected from the group consisting of Ezrin peptide 1 and the analogue of Ezrin peptide 1 of the formula (I) as defined in claim 1 and combinations thereof, and a pharmaceutically acceptable carrier for use in a method of treating post COVID-19 syndrome.

12. The pharmaceutical composition for use according to claim 11, which is formulated either for subcutaneous administration, oral administration, inhalation or intranasal administration.

## Patentansprüche

1. Verbindung, ausgewählt aus der Gruppe bestehend aus Ezrin-Peptid 1, das die Aminosäuresequenz NH2_Thr-Glu-Lys-Lys-Arg-Arg-Glu-Thr-Val-Glu-Arg-Glu-Lys-Glu_COOH (SEQ ID NO: 1) umfasst, dem Analogon von Ezrin-Peptid 1 der Formel (I) und Kombinationen aus Ezrin-Peptid 1 und dem Analogon von Ezrin-Peptid 1 der Formel (I),
NH2_X¹-Glu-Lys-Lys-Arg-Arg-Glu-Thr-Val-Glu-Arg-Glu-X²-X³_COOH (I)
wobei X¹, X² und X³ identisch oder unterschiedlich sind und unpolare Aminosäurereste sind, wobei die Aminosäuren insbesondere ausgewählt sind aus der Gruppe bestehend aus Glycin, Alanin, Valin, Leucin, Methionin, Isoleucin, Prolin, Phenylalanin, Tryptophan und Kombinationen davon,
zur Verwendung in einem Verfahren zur Behandlung des Post-COVID-19-Syndroms bei einer Person.

2. Verbindung zur Verwendung gemäß Anspruch 1, welche für die subkutane Verabreichung formuliert ist.

3. Verbindung zur Verwendung gemäß Anspruch 1, welche zur oralen Verabreichung formuliert ist.

4. Verbindung zur Verwendung gemäß Anspruch 1, welche zur Inhalation oder zur intranasalen Verabreichung formuliert ist.

5. Verbindung zur Verwendung gemäß einem der Ansprüche 1 bis 4, welche täglich verabreicht wird.

6. Verbindung zur Verwendung gemäß einem der Ansprüche 1 bis 5, welche in einer Dosierung von mindestens 0,05 mg pro Tag verabreicht wird.

7. Verbindung zur Verwendung gemäß einem der Ansprüche 1 bis 6, welche über einen Zeitraum von mindestens 5 Tagen in einer Dosierung im Bereich von 0,05 bis 5 mg pro Tag verabreicht wird.

8. Verbindung zur Anwendung gemäß einem der Ansprüche 1 bis 7, bei der es sich um Ezrin-Peptid 1, umfassend die AminosäuresequenzNH2_Thr-Glu-Lys-Lys-Arg-Arg-Glu-Thr-Val-Glu-Arg-Glu-Lys-Glu_COOH (SEQ ID NO:1), handelt.

9. Verbindung zur Verwendung gemäß einem der Ansprüche 1 bis 7, bei der es sich um das Analogon von Ezrin-Peptid 1 der Formel (I), wie in Anspruch 1 definiert, handelt.

10. Verbindung zur Verwendung gemäß einem der Ansprüche 1 bis 7, bei der es sich um eine Kombination aus Ezrin-Peptid 1 und dem Analogon von Ezrin-Peptid 1 der Formel (I), wie in Anspruch 1 definiert, handelt.

11. Pharmazeutische Zusammensetzung, umfassend mindestens eine Verbindung, ausgewählt aus der Gruppe bestehend aus Ezrin-Peptid 1 und dem Analogon von Ezrin-Peptid 1 der Formel (I), wie in Anspruch 1 definiert, und Kombinationen davon, und einen pharmazeutisch geeigneten Träger, zur Verwendung in einem Verfahren zur Behandlung des Post-COVID-19-Syndroms.

12. Pharmazeutische Zusammensetzung zur Verwendung gemäß Anspruch 11, welche entweder für die subkutane Verabreichung, die orale Verabreichung, die Inhalation oder die intranasale Verabreichung formuliert ist.

## Revendications

1. Composé choisi dans le groupe constitué du peptide Ezrine 1 comprenant la séquence d'acides aminés NH2_Thr-Glu-Lys-Lys-Arg-Arg-Glu-Thr-Val-Glu-Arg-Glu-Lys-Glu_COOH (SEQ ID NO:1), de l'analogue du peptide Ezrine 1 de formule (I) et des combinaisons du peptide Ezrine 1 et de l'analogue du peptide Ezrine 1 de formule (I),
NH2_X¹-Glu-Lys-Lys-Arg-Arg-Glu-Thr-Val-Glu-Arg-Glu-X²-X³_COOH (I)
où X¹, X² et X³ sont identiques ou différents et sont des résidus d'acides aminés non polaires, où les acides aminés sont en particulier choisis dans le groupe constitué de glycine, alanine, valine, leucine, méthionine, isoleucine, proline, phénylalanine, tryptophane et des combinaisons de ceux-ci,
pour une utilisation dans un procédé de traitement du syndrome post-COVID-19 chez un sujet.

2. Composé destiné à être utilisé selon la revendication 1, qui est formulé pour une administration sous-cutanée.

3. Composé destiné à être utilisé selon la revendication 1, qui est formulé pour une administration orale.

4. Composé destiné à être utilisé selon la revendication 1, qui est formulé pour une inhalation ou pour une administration intranasale.

5. Composé destiné à être utilisé selon l'une quelconque des revendications 1 à 4, qui est administré quotidiennement.

6. Composé destiné à être utilisé selon l'une quelconque des revendications 1 à 5, qui est administré à une dose d'au moins 0,05 mg par jour.

7. Composé destiné à être utilisé selon l'une quelconque des revendications 1 à 6, qui est administré pendant une période d'au moins 5 jours à une dose dans la plage de 0,05 à 5 mg par jour.

8. Composé destiné à être utilisé selon l'une quelconque des revendications 1 à 7, qui est le peptide Ezrine 1 comprenant la séquence d'acides aminés NH2_Thr-Glu-Lys-Lys-Arg-Arg-Glu-Thr-Val-Glu-Arg-Glu-Lys-Glu_COOH (SEQ ID NO:1).

9. Composé destiné à être utilisé selon l'une quelconque des revendications 1 à 7, qui est l'analogue du peptide Ezrine 1 de formule (I) tel que défini dans la revendication 1.

10. Composé destiné à être utilisé selon l'une quelconque des revendications 1 à 7, qui est une combinaison du peptide 1 d'Ezrine et de l'analogue du peptide 1 d'Ezrine de formule (I) tel que défini dans la revendication 1.

11. Composition pharmaceutique comprenant au moins un composé choisi dans le groupe constitué du peptide Ezrine 1 et de l'analogue du peptide Ezrine 1 de formule (I) tel que défini dans la revendication 1 et des combinaisons de ceux-ci, et un support pharmaceutiquement acceptable destiné à être utilisé dans un procédé de traitement du syndrome post-COVID-19.

12. Composition pharmaceutique destinée à être utilisée selon la revendication 11, qui est formulée soit pour une administration sous-cutanée, soit pour une administration orale, soit pour une inhalation, soit pour une administration intranasale.
